(19) Europäisches Patentamt
European Patent Office
Office européen des brevets

(11) Publication number: **0 074 837**
**A1**

(12) # EUROPEAN PATENT APPLICATION

(21) Application number: 82304810.3

(22) Date of filing: 13.09.82

(51) Int. Cl.³: C 07 D 401/04
C 07 D 213/74, C 07 B 27/00
B 01 J 31/02

(30) Priority: 11.09.81 US 301311
22.04.82 US 370725

(43) Date of publication of application:
23.03.83 Bulletin 83/12

(84) Designated Contracting States:
BE CH DE FR GB IT LI NL

(71) Applicant: REILLY TAR & CHEMICAL CORPORATION
1510 Market Square Center
Indianapolis Indiana 46204(US)

(72) Inventor: Scriven, Eric F. V.
8809 Meadowridge Lane
Indianapolis Indiana 46217(US)

(72) Inventor: Huckstep, L. Mark
412 Swallow Lane
Plainfield Indiana 46168(US)

(74) Representative: Bannerman, David Gardner et al,
Withers & Rogers 4 Dyer's Buildings Holborn
London, EC1N 2JT(GB)

(54) **4-Substituted pyridine catalysts.**

(57) As a new composition of matter, a composition selected from the group consisting of
4-(4'-methylpiperidino)pyridine,
4-hexamethyleneiminopyridine,
4-(4'-ethylpiperidino)pyridine and
4-(4'-methylpiperazino)pyridine,
and its preparation and utility as a liquid catalyst for acylation-type and alkylation-type reactions.

EP 0 074 837 A1

# 4-SUBSTITUTED PYRIDINE CATALYSTS

## Background of the Invention

This invention relates generally to the field of pyridine chemistry, and more particularly to certain pyridine derivatives which have shown specific utility as catalysts in acylations and alkylations and related reactions.

The work of catalysts is well documented. The ability of such substances to bring about a change in the rate of a chemical reaction without themselves being consumed or changing their own chemical identity is highly beneficial in both laboratory and commercial applications. In pyridine chemistry, pyridine itself as the parent compound to a large family of homologues and other derivatives has been shown to function as a catalyst and solvent for certain types of reactions, although reaction times have often been long and coupled with low yields. Typical of these are "acylation-type" reactions which as used in this application is understood to mean not only what may traditionally be termed an acylation, in which an acyl radical is incorporated into an organic molecule by substitution, but also recognized related reactions such as, for example, formylations, carbamoylations, phosphorylations, arylsulfonylations, lactonizations, cyanylations, esterifications and others. Also typical are "alkylation-type" reactions which as used in this

application is understood to mean an alkylation in the classical sense of introducing an alkyl radical into an organic compound by substitution or addition, as well as recognized related reactions such as, for example, trialkylsilylations, tritylations and others.

With these acylation-type and alkylation-type reactions, as with other reactions in general, the search for more efficient, chemically reactive catalysts is a continuous process. In this search, 4-dimethylaminopyridine has been found to effectively catalyze acylations and alkylations as well as several similar reactions. G. Hoefle, W. Steglich and H. Vorbrueggen, Angew. Chem. Int. Ed. Engl., 17, 569 (1978). It has been reported that 4-dimethylaminopyridine is approximately $10^4$ times more active than pyridine itself which was formerly used as a catalyst and solvent, as mentioned above. Referring to specific reactions, for example, tertiary alcohols are notoriously difficult to acylate cleanly and in high yield owing to steric hindrance. However, treatment of linalool with acetic anhydride and 4-dimethylaminopyridine in triethylamine was reported to give an 80% yield of linalyl acetate after standing for 14 hours at 24°C. Mesitol, a sterically hindered phenol more accurately identified as 2,4,6-trimethylphenol, has also been reported as being acylated smoothly in high yield by acetic anhydride in the presence of 4-dimethylaminopyridine. G. Hoefle and W. Steglich, Synthesis, 619 (1972).

The versatility and power of 4-dimethylaminopyridine as a catalyst are best exemplified by consideration of its use in processes of particular commercial importance. For example, 4-dimethylaminopyridine has been claimed to accelerate the formation of polyurethanes from p-toluene-diisocyanate and glycols (British Patents Nos. 990,635 and 990,633) as well as the formation of polyepoxides (British Patent No. 1,201,756) and polyamides

(British Patent No. 1,207,673). Production of an ampicillin by treatment of 6-isocyanatopenicillinate with D-N-benzyloxycarbonyl-2-phenylglycine was also reported as catalyzed by 4-dimethylaminopyridine in German Offen. No. 2,155,152. Commercial production was also reported of phosphorothioate esters with insecticidal and acaricidal activity of pyridines, pyrazines (Belgian Patent No. 832,047) and pyrimidines (British Patent No. 2,011,415).

Notwithstanding these advantages experienced with 4-dimethylaminopyridine as a catalyst, disadvantages have also been encountered with its use. One disadvantage is that the compound is a solid. This causes problems both with laboratory use and particularly with industrial applications where the procedure for adding a solid to a batch or continuous reaction can be difficult. Consequently, the compound would generally be used with a co-solvent in such circumstances to lessen at least some of this problem. However, difficulty is then often experienced in separating out the desired product, especially if basic, from the catalyst when working up the reaction. Moreover, recovery of the 4-dimethylaminopyridine catalyst itself can often prove to be extremely troublesome under such conditions. These difficulties would not exist with a liquid catalyst that has the same efficacy as 4-dimethylaminopyridine in reactions such as the above examples. No such catalyst was known to applicants prior to their present discovery. While it is true that pyridine is a liquid, it is far less effective a catalyst as mentioned above.

## Summary of the Invention

With this background, one aspect of the present invention comprises as a new composition of matter, a composition selected from the group consisting of 4-(4'-methylpiperidino)pyridine, 4-hexamethyleneiminopyridine, 4-(4'-ethylpiperidino)pyridine and 4-(4'-methylpiperazino)pyridine, and having the respective formulas as set forth in the Description section to follow.

The above compositions are liquid and have shown good catalytic ability in acylation-type and alkylation-type reactions far superior to pyridine and at least comparable to 4-dimethylaminopyridine, but without the disadvantages encountered with these compounds. Preliminary investigations have also been encouraging in evidencing potential for further uses of applicants' compositions in both catalytic and possibly other applications. In addition, applicants are hopeful that further testing will uncover other liquid compositions within this same family which will prove to have similar advantages in catalytic and other uses.

## Description of the Preferred Embodiment

As mentioned above, applicants have discovered new compositions of matter which are identified as follows:

4-(4'-methylpiperidino)pyridine having the formula;

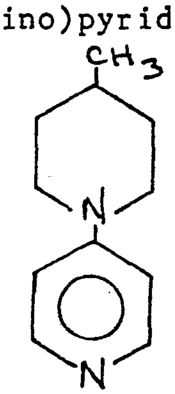

4-hexamethyleneiminopyridine having the formula;

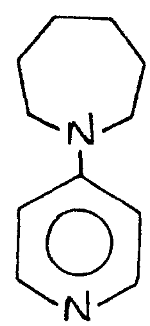

4-(4'-ethylpiperidino)pyridine having the formula;

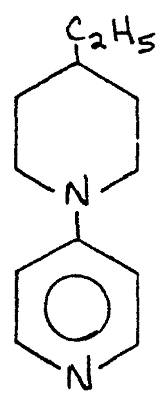

and 4-(4'-methylpiperazino)pyridine having the formula.

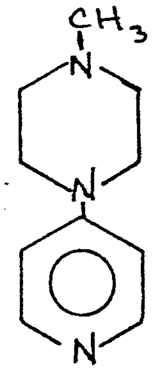

These compositions are liquid and have been isolated and identified as having boiling points in the range of 155-175°C at pressures in the range of 2-5.5 mm Hg as indicated for each composition in the specific examples below.

As for utility, each of applicants' compositions have proven themselves far superior to pyridine and at least comparable to 4-dimethylaminopyridine as catalysts in acylation-type and alkylation-type reactions. Quantitative comparison of the catalytic ability of each of applicants' compounds with 4-dimethylaminopyridine was accomplished using the method disclosed in Connors and Albert, J. Pharm. Science, 62, 845 (1973). Under standard conditions, for example, progress of the acylation of isopropanol with acetic anhydride at 40°C after 5 minutes was 92% in the presence of 4-dimethylaminopyridine and 88% in the presence of 4-(4'-methylpiperidino)pyridine. As a second example, the acylation of the sterically hindered alcohol t-butanol with acetic anhydride at 69°C for a period of 2 hours was 38% complete using 4-dimethylaminopyridine as catalyst. This was compared to 37% acylation under the same conditions using applicants' 4-(4'-methylpiperidino)pyridine. As a third example, the tritylation, or as it is more completely termed the triphenylmethylation, of benzyl alcohol using applicants' 4-(4'-methylpiperidino)pyridine catalyst yielded 89% product after being allowed to stand overnight at room

temperature under nitrogen. Similar results at least comparable to those achieved with 4-dimethylaminopyridine have been obtained using each of applicants' other three compositions.

In each reaction tested to date, applicants' catalysts have functioned effectively without any attendant complications from the use of a co-solvent or other additive or from reisolation and recovery of the catalyst after the reaction was complete. From these successes, it is reasonable to expect that each of applicants' compositions will function equally well as a catalyst in other acylation-type and alkylation-type reactions such as those mentioned earlier in the application.

For the purpose of promoting a better understanding of the disclosure herein and the nature and scope of applicants' discovery, the following specific examples are given of a method of preparing each of applicants' compositions and then using each as a catalyst in an acylation-type and an alkylation-type reaction.

## Example 1

To 31.5 parts of 4-cyanopyridine was added 25 parts of water, 44 parts of concentrated hydrochloric acid and 21 parts of 2-vinylpyridine. The resulting mixture was heated at 60°C for 6 hours and cooled to 30°C, at which time 36.5 parts of 4-methylpiperidine was added. The mixture was then stirred 2 hours at 30°C, at which time 200 parts of 40% sodium hydroxide was added and the mixture boiled under reflux for an additional 2-hour period. The solution was then cooled, and the organic layer separated and distilled to give 35 parts of 4-(4'-methylpiperidino)pyridine which was present as a liquid having a boiling point of 163°C at 5.5 mm Hg.

The preparation of 2,4,6-trimethylphenyl acetate was later accomplished by slowly adding acetic anhyride (15 g, 0.15 moles) to a solution of 2,4,6,trimethylphenol (13.6 g

0.1 mole) and 4-(4'-methylpiperidino)pyridine (8.5 g, 0.05 mole) in methylene chloride (100 ml). After allowing the reaction mixture to stand for 2 hours, diethyl ether was added and the ethereal solution was washed with dilute hydrochloric acid and then saturated sodium bicarbonate solution. The extract was dried ($MgSO_4$), the solvent was removed and the residue was distilled to give 2,4,6-trimethylphenyl acetate compound (15.5 g, 87%) with a boiling point of 64°C at 0.5 mm Hg. The 4-(4'-methylpiperidino)pyridine catalyst was recovered in the dilute hydrochloric acid and reisolated by standard neutralization, extraction and distillation procedures.

The preparation of triphenylmethylbenzyl ether was also later accomplished by allowing a mixture of benzyl alcohol (11 g, 0.1 mole), triphenylmethyl chloride (33.4 g, 0.12 mole), 4-(4'-methylpiperidino)pyridine (1 g, 0.006 moles) and triethylamine (25 ml) in methylene chloride to stand overnight at room temperature under nitrogen. The reaction mixture was evaporated the next day almost to dryness and the residue was crystallized from ethanol to yield triphenylmethylbenzyl ether (31 g, 89%) with a melting point of 95°C. The 4-(4'-methylpiperidino)pyridine catalyst remained unchanged and was reisolated by standard neutralization, extraction and distillation procedures.

<u>Example</u> 2

To 31.5 parts of 4-cyanopyridine was added 25 parts of water, 44 parts of concentrated hydrochloric acid and 21 parts of 2-vinylpyridine. The resulting mixture was heated at 60°C for 6 hours and cooled to 30°C, at which time 36.5 parts of 4-ethylpiperidine was added. The mixture was then stirred 2 hours at 30°C, at which time 200 parts of 40% sodium hydroxide was added and the mixture boiled under reflux for an additional 2-hour period. The solution was then cooled, and the organic

layer separated and distilled to give 36 parts of 4-(4'-ethylpiperidino)pyridine which was present as a liquid having a boiling point of 167-8°C at 4 mm Hg.

The preparation of 2,4,6-trimethylphenyl acetate was later accomplished by slowly adding acetic anhyride (15 g, 0.15 moles) to a solution of 2,4,6,trimethylphenol (13.6 g 0.1 mole) and 4-(4'-ethylpiperidino)pyridine (9.2 g, 0.05 mole) in methylene chloride (100 ml). After allowing the reaction mixture to stand for 2 hours, diethyl ether was added and the ethereal solution was washed with dilute hydrochloric acid and then saturated sodium bicarbonate solution. The extract was dried ($MgSO_4$), the solvent was removed and the residue was distilled to give 2,4,6-trimethylphenyl acetate compound (17.25 g, 97%) with a boiling point of 103-105°C at 4.0 mm Hg. The 4-(4'-ethylpiperidino)pyridine catalyst was recovered in the dilute hydrochloric acid and reisolated by standard neutralization, extraction and distillation procedures.

The preparation of triphenylmethylbenzyl ether was also later accomplished by allowing a mixture of benzyl alcohol (2.2 g, 0.02 mole), triphenylmethyl chloride (5.9 g, 0.021 mole), 4-(4'-ethylpiperidino)pyridine (0.15 g, 0.8 mmole) and triethylamine (7 ml) in methylene chloride to stand overnight at room temperature under nitrogen. The reaction mixture was evaporated the next day almost to dryness and the residue was crystallized from ethanol to yield triphenylmethylbenzyl ether (6.25 g, 90%) with a melting point of 95°C. The 4-(4'-ethylpiperidino)pyridine catalyst remained unchanged and was reisolated by standard neutralization, extraction and distillation procedures.

Example 3

To 31.5 parts of 4-cyanopyridine was added 25 parts of water, 44 parts of concentrated hydrochloric acid and 21 parts of 2-vinylpyridine. The resulting mixture was heated at 60°C for 6 hours and cooled to 30°C, at which

time 36.5 parts of N-methylpiperazine was added. The mixture was then stirred 2 hours at 30°C, at which time 200 parts of 40% sodium hydroxide was added and the mixture boiled under reflux for an additional 2-hour period. The solution was then cooled, and the organic layer separated and distilled to give 37 parts of 4-(4'-methylpiperazino)pyridine which was present as a liquid having a boiling point of 155-165°C at 4 mm Hg.

The preparation of 2,4,6-trimethylphenyl acetate was later accomplished by slowly adding acetic anhyride (15 g, 0.15 moles) to a solution of 2,4,6,trimethylphenol (13.6 g 0.1 mole) and 4-(4'-methylpiperazino)pyridine (8.85 g, 0.05 mole) in methylene chloride (100 ml). After allowing the reaction mixture to stand for 2 hours, diethyl ether was added and the ethereal solution was washed with dilute hydrochloric acid and then saturated sodium bicarbonate solution. The extract was dried ($MgSO_4$), the solvent was removed and the residue was distilled to give 2,4,6-trimethylphenyl acetate compound (13.5 g, 76%) with a boiling point of 103-5°C at 4.0 mm Hg. The 4-(4'-methylpiperazino)pyridine catalyst was recovered in the dilute hydrochloric acid and reisolated by standard neutralization, extraction and distillation procedures.

The preparation of triphenylmethylbenzyl ether was also later accomplished by allowing a mixture of benzyl alcohol (2.2 g, 0.02 mole), triphenylmethyl chloride (5.9 g, 0.21 mole), 4-(4'-methylpiperazino)pyridine (0.14 g, 0.8 mmoles) and triethylamine (7 ml) in methylene chloride to stand overnight at room temperature under nitrogen. The reaction mixture was evaporated the next day almost to dryness and the residue was crystallized from ethanol to yield triphenylmethylbenzyl ether (5.5 g, 78%) with a melting point of 95°C. The 4-(4'-methylpiperazino)pyridine catalyst remained unchanged and was reisolated by standard neutralization, extraction and distillation procedures.

Example 4

To 31.5 parts of 4-cyanopyridine was added 25 parts of water, 44 parts of concentrated hydrochloric acid and 21 parts of 2-vinylpyridine. The resulting mixture was heated at 60°C for 6 hours and cooled to 30°C, at which time 36.5 parts of hexahydroazepine was added. The mixture was then stirred 2 hours at 30°C, at which time 200 parts of 40% sodium hydroxide was added and the mixture boiled under reflux for an additional 2-hour period. The solution was then cooled, and the organic layer separated and distilled to give 35.5 parts of 4-hexamethyleneiminopyridine which was present as a liquid having a boiling point of 160-175°C at 2 mm Hg.

The preparation of 2,4,6-trimethylphenyl acetate was later accomplished by slowly adding acetic anhyride (15 g, 0.15 moles) to a solution of 2,4,6,trimethylphenol (13.6 g 0.1 mole) and 4-hexamethyleneiminopyridine (8.8 g, 0.05 mole) in methylene chloride (100 ml). After allowing the reaction mixture to stand for 2 hours, diethyl ether was added and the ethereal solution was washed with dilute hydrochloric acid and then saturated sodium bicarbonate solution. The extract was dried ($MgSO_4$), the solvent was removed and the residue was distilled to give 2,4,6-trimethylphenyl acetate compound (16.9 g, 95%) with a boiling point of 103-105°C at 4.0 mm Hg. The 4-hexamethyleneiminopyridine catalyst was recovered in the dilute hydrochloric acid and reisolated by standard neutralization, extraction and distillation procedures.

The preparation of triphenylmethylbenzyl ether was also later accomplished by allowing a mixture of benzyl alcohol (2.2 g, 0.02 mole), triphenylmethyl chloride (5.9 g, 0.21 mole), 4-hexamethyleneiminopyridine (0.14 g, 0.8 mmoles) and triethylamine (7 ml) in methylene chloride to stand overnight at room temperature under nitrogen. The reaction mixture was evaporated the next day almost to dryness and the residue was crystallized from ethanol to

yield triphenylmethylbenzyl ether (6.1 g, 87%) with a melting point of 95°C. The 4-hexamethyleneiminopyridine catalyst remained unchanged and was reisolated by standard neutralization, extraction and distillation procedures.

WE CLAIM:

1. As a new composition of matter, a composition selected from the group consisting of: 4-(4'-methylpiperidino)pyridine having the formula;

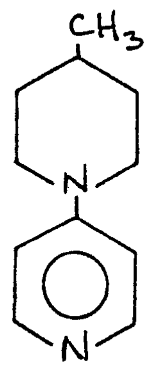

4-(4'-ethylpiperidino)pyridine having the formula;

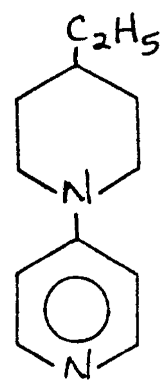

and 4-(4'-methylpiperazino)pyridine having the formula

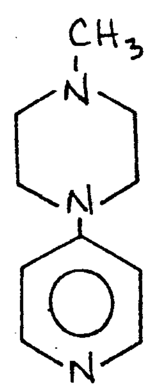

2. The composition in claim 1 in which said composition is 4-(4'-methylpiperidino)pyridine.

3. The composition in claim 1 in which said composition is 4-(4'-ethylpiperidino)pyridine.

4. The composition in claim 1 in which said composition is 4-(4'-methylpiperazino)pyridine.

5. In an acylation-type or alkylation-type reaction, the improvement comprising using a liquid catalyst selected from the group consisting of
4-(4'-methylpiperidino)pyridine,
4-hexamethyleneiminopyridine,
4-(4'-ethylpiperidino)pyridine, and
4-(4'-methylpiperazino)pyridine as a catalyst for the reaction.

6. The reaction of claim 5 in which said using is of 4-(4'-methylpiperidino)pyridine.

7. The reaction of claim 5 in which said using is of 4-hexamethyleneiminopyridine.

8. The reaction of claim 5 in which said using is of 4-(4'-ethylpiperidino)pyridine.

9. The reaction of claim 5 in which said using is of 4-(4'-methylpiperazino)pyridine.

10. The reaction of claim 5 comprising the additional step of recovering the catalyst after the reaction.

11. The reaction of claim 5 in which the reaction is an acylation-type reaction.

12. The reaction of claim 5 in which the reaction is an alkylation-type reaction.

13. The reaction of claim 5 in which said using is in the absence of a co-solvent.

European Patent Office

# EUROPEAN SEARCH REPORT

0074837

Application number

EP 82 30 4810.3

## DOCUMENTS CONSIDERED TO BE RELEVANT

| Category | Citation of document with indication, where appropriate, of relevant passages | Relevant to claim |
|---|---|---|
| A | US - A - 4 140 853 (SCHERING AG)<br>* column 1, lines 1 to 37,<br>column 2, lines 38 to 54 *<br>-- | 5-9,<br>11 |
| D,A | SYNTHESIS, November 1972, Stuttgart<br>G. HÖFLE et al. "4-Dialkylaminopyridines as Acylation Catalysts; III. Acylation of Sterically Hindered Alcohols"<br>pages 619 to 621<br>---- | |

CLASSIFICATION OF THE APPLICATION (Int. Cl. 3)

C 07 D 401/04
C 07 D 213/74
C 07 B 27/00
B 01 J 31/02

TECHNICAL FIELDS SEARCHED (Int.Cl. 3)

C 07 B 27/00
C 07 D 213/74
C 07 D 401/04

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 02-12-1982 | PHILLIPS |

EPO Form 1503.1 06.78